# EUROPÄISCHE PATENTSCHRIFT

(11) **EP 2 452 198 B1**
(45) Veröffentlichungstag und Bekanntmachung des Hinweises auf die Patenterteilung: **08.06.2016**
(21) Anmeldenummer: 10731720.8
(22) Anmeldetag: 06.07.2010
(51) Int. Cl.: G01N 33/543

(54) **DETEKTION VON ANTIGENEN**
DETECTION OF ANTIGENS
DÉTECTION D'ANTIGÈNES

(30) Priorität: 09.07.2009 DE 102009032502
(43) Veröffentlichungstag der Anmeldung: 16.05.2012
(73) Patentinhaber: Speetect GmbH, 82024 Taufkirchen (DE)
(72) Erfinder: REIDT, Ulrich, 34613 Schwalmstadt (DE); FRIEDBERGER, Alois, 85667 Oberpframmem (DE); HELLER, Christoph, 82024 Taufkirchen (DE); BRAHMS, Hero, 35037 Marburg (DE)
(74) Vertreter: Krebs, Jörg
(86) Internationale Anmeldenummer: PCT/EP2010/004128
(87) Internationale Veröffentlichungsnummer: WO 2011/003598

(56) Entgegenhaltungen:
- WO-A2-2004/057021
- ENGELHART S ET AL: "Hospital-acquired legionellosis originating from a cooling tower during a period of thermal inversion" INTERNATIONAL JOURNAL OF HYGIENE AND ENVIRONMENTAL HEALTH, Bd. 211, Nr. 3-4, 15. Juli 2008 (2008-07-15), Seiten 235-240, XP022736319 URBAN U. FISCHER, JENA, DE ISSN: 1438-4639 DOI: 10.1016/J.IJHEH.2007.07.022 [gefunden am 2008-06-17]
- AOYAMA ET AL: "The actin-related protein hArp8 accumulates on the mitotic chromosomes and functions in chromosome alignment" EXPERIMENTAL CELL RESEARCH, Bd. 314, Nr. 4, 4. Dezember 2007 (2007-12-04), Seiten 859-868, XP022461345 ACADEMIC PRESS, US ISSN: 0014-4827 DOI: 10.1016/J.YEXCR.2007.11.020
- HOFFMAN P S ET AL: "Cloning and nucleotide sequence of a gene (ompS) encoding the major outer membrane protein of Legionella pneumophila." JOURNAL OF BACTERIOLOGY, Bd. 174, Nr. 3, Februar 1992 (1992-02), Seiten 914-920, XP002608206 ISSN: 0021-9193
- DIAS NETO E ET AL: "Shotgun sequencing of the human transcriptome with ORF expressed sequence tags." PROCEEDINGS OF THE NATIONAL ACADEMY OF SCIENCES OF THE UNITED STATES OF AMERICA, Bd. 97, Nr. 7, 28. März 2000 (2000-03-28), Seiten 3491-3496, XP002608207 ISSN: 0027-8424 -& DATABASE EMBL [Online] 28. September 2004 (2004-09-28), XP002612954 gefunden im EBI Database accession no. CV375551
- POUSTKA ALBERT J ET AL: "Generation, annotation, evolutionary analysis, and database integration of 20,000 unique sea urchin EST clusters." GENOME RESEARCH, Bd. 13, Nr. 12, Dezember 2003 (2003-12), Seiten 2736-2746, XP002608208 ISSN: 1088-9051 -& DATABASE EMBL [Online] 18. September 2003 (2003-09-18), XP002612955 gefunden im EBI Database accession no. CD339852
- H Yu ET AL: "Detection of biological threat agents by immunomagnetic microsphere-based solid phase fluorogenic- and electro-chemiluminescence", Biosensors and Bioelectronics, vol. 14, no. 10-11, 1 January 2000 (2000-01-01), pages 829-840, XP055162449, ISSN: 0956-5663, DOI: 10.1016/S0956-5663(99)00068-8
- Sverre-Henning Brorson: "Bovine serum albumin (BSA) as a reagent against non-specific immunogold labeling on LR-White and epoxy resin", Micron, vol. 28, no. 3, 1 June 1997 (1997-06-01), pages 189-195, XP055192337, ISSN: 0968-4328, DOI: 10.1016/S0968-4328(97)00030-9

SEQUENCE LISTING
<110> EADS Deutschland GmbH
<120> Detektion von Antigenen
<130> 1109499
<160> 4
<170> PatentIn Version 3.3
<210> 1
   <211> 22
   <212> DNA
   <213> artificial
<220>
   <223> Forward Primer für das Legionella pneumophila ompS Gen
<400> 1
   gcggctgtat ttgctctggg aa 22
<210> 2
   <211> 24
   <212> DNA
   <213> artificial
<220>
   <223> Reverse Primer für das Legionella pneumophila omps Gen
<400> 2
   taagcctatg taggggccag atgc 24
<210> 3
   <211> 20
   <212> DNA
   <213> artificial
<220>
   <223> Forward Primer für das adenovirale Hexon-Gen
<400> 3
   gaaatgacac caacgaccag 20
<210> 4
   <211> 20
   <212> DNA
   <213> artificial
<220>
   <223> Reverse Primer für das adenovirale Hexon Gen
<400> 4
   gggaacatta gcggggtaag 20

## Beschreibung

### GEBIET DER ERFINDUNG

Die vorliegende Erfindung liegt auf dem Gebiet der Detektion von Pathogenen und Toxinen in Proben unter Verwendung von mit Antikörpern beschichteten magnetischen Beads.

### HINTERGRUND DER ERFINDUNG

Die Detektion von Pathogenen oder Toxinen in Proben ist von hoher Relevanz, z.B. für klinische oder für Sicherheitsanwendungen. So ist es häufig wünschenswert innerhalb einer kurzen Zeitdauer verlässlich ein bestimmtes Pathogen und/oder Toxin in einer Probe nachweisen zu können.

Im Stand der Technik sind eine Vielzahl von Nachweisverfahren bekannt, die auf dem Einsatz von gegen das Pathogen und/oder Toxin gerichteten Antikörpern basieren. Antikörper werden deshalb häufig eingesetzt, da sie in der Regel eine relativ hohe Bindekonstante für ihr Antigen aufweisen uns somit eine hohe Spezifität erreichen. Desweiteren können Antikörper für eine fast beliebige Anzahl von Antigenen, d.h. z.B. Pathogene oder Toxine erzeugt werden.

Problematisch bei der Verwendung von Antikörpern ist dennoch immer eine Kreuzreaktivität, d.h. eine unspezifische Bindung an unerwünschte Ziele. Dementsprechend treten bei der Verwendung von Antikörper-basierten Nachweisverfahren immer auch Bindungsreaktionen der Antikörper an Moleküle auf, die nicht den eigentlichen Antigenen der Antikörper entsprechen. Somit kommt es häufig zur Detektion von sogenannten falsch-positiven Antigenen. Außerdem wird auch häufig eine Bindung von Antigenen an das Substrat beobachtet, die nicht den zu detektierenden Antigenen entsprechen, jedoch durch das nachgeschaltete Detektionsverfahren detektiert werden. Beispielsweise binden bei einer Kontaktierung von Antikörper-beschichteten magnetischen Beads mit einer Probe immer auch in der Probe befindliche Antigene, wie beispielsweise phylogenetisch verwandte Pathogene, an die Beads selber und nicht an die daran gebundenen Antikörper. In der nachgeschalteten Detektionsreaktion werden dann diese, eigentlich nicht zu detektierenden, Antigene teilweise ebenfalls als "Falsch-positive" ermittelt oder erschweren als "Hintergrund" den Nachweis der eigentlich zu detektierenden Antigene.

H Yu et al "Detection of biologocal threat agents by immunimagnetic microsphere-based solid phase fluororganic- and electrochemiluminescence", Biosensors and Biolelectronics, Bd. 14, Nr. 10-11, 1. Januar 2000, Seiten 829-840 offenbart beispielsweise eine Methode zur magnetischen Immunoseparierung unter der Verwendung einer Biotin-Streptavidin Kopplung.

### ZUSAMMENFASSUNG DER ERFINDUNG

Es ist die Aufgabe der vorliegenden Erfindung ein hochspezifisches und verlässliches auf Antikörpern basierendes Nachweisverfahren für die Detektion von Pathogenen und/oder Toxinen bereitzustellen. Eine weitere Aufgabe der vorliegenden Erfindung liegt in der Bereitstellung eines besonderen Waschpuffers, der innerhalb dieses Verfahrens verwendet werden kann, spezifischer Primersequenzen zur Detektion bestimmter Pathogene, sowie Kits und Vorrichtungen zur Durchführung des erfindungsgemäßen Verfahrens.

Bezüglich des Nachweisverfahrens wird die Aufgabe mittels des Verfahrens nach Anspruch 1 gelöst. Insbesondere wird ein Verfahren zur Detektion zumindest eines Antigens bereitgestellt, welches die Schritte: a) Bereitstellen von magnetischen Beads, die mit spezifischen Antikörpern für zumindest ein zu detektierendes Antigen beschichtet sind; b) Kontaktieren der magnetischen Beads mit einem Waschpuffer, der zumindest 8 % BSA umfasst; c) Inkubation des Ansatzes aus Schritt b) mit einer Probe; d) Isolierung der magnetischen Beads mittels eines magnetischen Separators; und e) Detektion der mittels der Antikörper an die magnetischen Beads gebundenen Antigene umfasst. Bezüglich des Waschpuffers, der Primersequenzen, der Kits und Vorrichtungen wird die Aufgabe gemäß der Ansprüche 8, 9, 10 bzw. 11 gelöst.

Der vorliegenden Erfindung liegt die überraschende Erkenntnis zu Grunde, dass die Verwendung eines Waschpuffers, der zumindest 8 % und bevorzugt 10 % BSA enthält, eine hochspezifische Bindung der verwendeten Antikörper an ihre Antigene ermöglicht, d.h. sowohl die Anzahl der Falsch-positiven als auch die Hintergrundreaktion signifikant erniedrigt.

Das Verfahren der vorliegenden Erfindung basiert auf Antikörper-beschichteten magnetischen Beads als zentrales Element. Magnetische Beads, im Folgenden kurz mit "Beads" bezeichnet, sind dem Fachmann bekannt und können beispielsweise von Invitrogen bezogen werden. Sie bestehen für gewöhnlich aus Polymerkügelchen, vorzugsweise bestehend aus Latex und/oder Polystyren, mit einem paramagnetischen Kern. Die Beads selbst haben vorzugsweise einen Durchmesser im Bereich von ungefähr 1 µm bis 50 µm. Dem Fachmann wird einleuchten, dass jedoch auch jedes andere geeignete Material und oder andere Größen der Beads verwendet werden können.

Die Beschichtung der Beads mit Antikörpern wird auch als "Coating" bezeichnet und kann auf mehrere dem Fachmann hinlänglich bekannte Verfahren erfolgen. Bevorzugte Verfahren zur Beschichtung der Beads mit Antikörpern schließen ein: a) Passive Adsorption, z.B. über hydrophobe Wechselwirkungen; b) direkte chemische Kopplung, auch als "Crosslinking" bekannt, vorzugsweise über eine Peptidbindung oder andere Bindungen; c) Kopplung über immobilisierte antikörperbindender Proteine, wie beispielsweise Protein A; d) Kopplung über eine Biotin-Streptavidin-Bindung.

In einer bevorzugten Ausführungsform erfolgt das Coating über eine Biotin-Streptavidin-Bindung, welche die hohe Affinität zweier spezieller biologischer Moleküle, nämlich Biotin und Streptavidin, zueinander ausnutzt. Hierzu wird der Antikörper an der seinen spezifischen Bindungsstellen abgewandten Seite, der sogenannten Fc-Domäne, mit dem Molekül Biotin markiert. Gleichzeitig wird das Protein Streptavidin auf die Oberfläche der Beads gekoppelt. Werden Antikörper und Beads nun miteinander vermischt, so werden die Antikörper vorteilhaft praktisch irreversibel an die Kugeloberfläche gebunden.

Ein weiterer Vorteil dieser Ausführungsform besteht darin, dass nur eine Sorte von Beads, nämlich Streptavidin-Beads hergestellt werden muss. Durch Kopplung mit biotinylierten Antikörpern können so einfach Beads mit fast jeder gewünschten Spezifität erzeugt werden.

Nach der Immobilisierung der Antikörper an die Beads können diese für das gezielte "Einfangen" der spezifischen Antigene, d.h. z.B. von Pathogenen und/oder Toxinen, eingesetzt werden.

Die Antikörper können gegen beliebige Antigene ausgewählt aus der Gruppe Legionellen, Adenoviren oder das E. coli Bakterium gerichtet sein.

Die Antikörper der vorliegenden Erfindung können entweder durch den Fachmann mittels hinlänglich bekannter Verfahren, beispielsweise mittels der Hybridomtechnik, bereitgestellt werden, oder kommerziell, beispielsweise von Acris Antibodies, Novus Biologicals und/oder Abcam bezogen werden.

In einer Ausführungsform ist der Antikörper ein polyklonaler Antikörper, bevorzugt ist er ein monoklonaler Antikörper. Dies hat den Vorteil, dass eine höhere Spezifität erreicht wird.

Ein Antikörper wird vorliegend als ein "spezifischer Antikörper" angesehen, wenn er eine ausreichend hohe Bindekonstante für sein Antigen aufweist, um eine Detektion des Antigens mit den Verfahren der vorliegenden Erfindung zu ermöglichen. Dies bedeutet, dass die Bindekonstante hinreichend groß sein muss, um das Zielmolekül zu komplexieren. In bevorzugten Ausführungsformen hat ein solcher spezifischer Antikörper eine Bindekonstante von ungefähr 10⁵ M⁻¹ bis ungefähr 10¹⁰ M⁻¹, bevorzugter 10⁸ M⁻¹ bis ungefähr 10¹¹ M⁻¹, noch bevorzugter 10⁹ M⁻¹ bis ungefähr 10¹² M⁻¹. Eine höhere Bindekonstante eines Antikörpers hat den Vorteil, dass das entsprechende Antigen mit höherer Spezifität und mit kürzerer Inkubationszeit detektiert werden kann.

In einer bevorzugten Ausführungsform umfassen die Beads mehr als einen spezifischen Antikörper. Dies bedeutet, dass entweder verschiedene Arten von Beads mit jeweils einem Antikörper und/oder eine Art von Beads mit jeweils zumindest zwei Antikörpern vorliegen. In einer noch bevorzugteren Ausführungsform liegen Antikörper für einen Mikroorganismus, d.h. ein Bakterium, Virus und/oder einen Pilz vor, und gleichzeitig Antikörper für ein von diesem Mikroorganismus produziertes Toxin. Dies hat den Vorteil, dass eine verbesserte Spezifität und/oder Sensitivität bei der Detektion erreicht wird, da neben dem zu detektierenden Mikroorganismus gleichzeitig immer auch ein von diesem produziertes Toxin detektiert wird.

Während bei den meisten konventionellen Nachweisverfahren die Fängerantikörper an eine feste Matrix gebunden sind, z.B. Mikrotiterplatten, Membranen, oder Säulen, bietet die erfindungsgemäße Verwendung magnetischer Beads mehr Flexibilität und macht den Test weitgehend unabhängig von einem bestimmten Format oder einer bestimmten Plattform.

In einem weiteren Schritt des erfindungsgenmäßen Verfahrens werden die Beads mit einem Waschpuffer kontaktiert. Für diese Kontaktierung der Beads kann jede geeignete Gefäßform, z.B. Mikrotiterplatte, Säule, Mikrokanäle, etc., verwendet werden.

Erfindungsgemäß werden die Beads hierbei mit einem Waschpuffer in Kontakt gebracht, der zumindest 8 % BSA, d.h. bovines Serum Albumin, umfasst. Vorzugsweise umfasst der Waschpuffer ≥ 8 % und ≤ 12 % BSA, noch bevorzugter umfasst der Waschpuffer ≥ 9 % und ≤ 10 % BSA, am bevorzugtesten umfasst der Waschpuffer eine BSA-Anteil von 10 %. Überraschenderweise zeigte sich, dass solche Konzentrationen von BSA eine hervorragende und signifikant bessere Detektion der Antigene erlaubt. Die Prozentangaben betreffen Gewichtsprozent. In einer weiteren bevorzugten Ausführungsform ist das BSA ein biotinfreies BSA.

In weiteren bevorzugten Ausführungsformen umfasst der Waschpuffer weiterhin zumindest ein Detergenz und Wasser, bevorzugt Aqua dest., zum Auffüllen. In einer weiteren bevorzugten Ausführungsform ist das Detergenz Tween 20 und/oder Triton-X und liegt noch bevorzugter in einer Konzentration von 0,01 Vol-% vor.

In weiteren bevorzugten Ausführungsformen umfasst der Waschpuffer weiterhin zumindest eine der folgenden Substanzen: Kaliumchlorid, Kaliumdihydrogenphosphat, Dinatriumhydrogenphosphat und/oder Natriumchlorid. Noch bevorzugter liegen diese Substanzen in folgenden Konzentrationen vor: Kaliumchlorid 2,7 mM, Kaliumdihydrogenphosphat 1,5 mM, Dinatriumhydrogenphosphat 8,1 mM und/oder Natriumchlorid 136,9 mM.

In einer weiteren bevorzugten Ausführungsform ist der Waschpuffer ein PBS Waschpuffer umfassend Kaliumchlorid, Kaliumdihydrogenphosphat, Dinatriumhydrogenphosphat und/oder Natriumchlorid und umfasst weiterhin BSA und bevorzugt zumindest ein Detergenz.

In einer weiteren bevorzugten Ausführungsform hat der Waschpuffer einen pH von ≥ 5 und ≤ 9, bevorzugter ≥ 6 und ≤ 8, noch bevorzugter ≥ 6,5 und ≤ 8 oder ≥ 7 und ≤ 8 und am bevorzugtesten von ungefähr 7,4. In einer weiteren Ausführungsform wird dieser pH-Wert des Waschpuffers mittels HCl oder NaOH eingestellt.

In einer weitem bevorzugten Ausführungsform werden die Beads für eine bestimmte Zeidauer mit dem Waschpuffer inkubiert. Vorzugsweise reicht diese Zeitdauer von ≥ 1 min und ≤ 24 h, ≥ 30 min und ≤ 12 h, ≥ 1 h und ≤ 6 h, ≥ 2 h und ≤ 8 h. Noch bevorzugter erfolgt die Inkubation über Nacht.

In einem weiteren Schritt des erfindungsgenmäßen Verfahrens werden die mit dem Waschpuffer kontaktierten Beads mit einer Probe inkubiert. Für diese Inkubation kann erneut jede geeignete Gefäßform, z.B. Mikrotiterplatte, Säule, Mikrokanäle, etc., verwendet werden.

Die Probe kann jegliche geeignete Probe sein, die ein zu detektierendes Antigen enthalten könnte. In bevorzugten Ausführungsformen ist die Probe eine flüssige, halbflüssige oder gasförmige Probe, eine Körperflüssigkeit, Blut, Speichel, Serum, Tränenflüssigkeit, Urin, eine Lebensmittelprobe, ein Abstrich, z.B. aus einem kritischen Gebiet oder Gebäude wie einem Gericht, einem Krankenhaus, einem Kriegsgebiet oder aus dem Weltraum. In weiteren Ausführungsformen entstammt die Probe aus einem Belüftungssystem, beispielsweise einer Klimaanlage, bevorzugter aus einem Belüftungssystem eines Krankenhauses, einer öffentlichen Einrichtung, oder eines Flughafens.

Die Inkubation erfolgt durch Inkontaktbringen der mit Waschpuffer kontaktierten Beads mit der Probe unter geeigneten Bedingungen, die eine Komplexierung des zumindest einen spezifischen Antikörpers auf den Beads mit seinem Antigen erlauben. In bevorzugten Ausführungsformen wird die Inkubation bei Raumtemperatur oder bei einer Temperatur zwischen ≥ 20° C und ≤ 40° C, ≥ 30° C und ≤ 40° C und noch bevorzugter bei etwa 37° C durchgeführt.

In einer weiteren bevorzugten Ausführungsform erfolgt die Inkubation für eine Zeitdauer von ≥ 1 min und ≤ 3 h, ≥ 10 min und ≤ 2 h, ≥ 20 min und ≤ 2 h, ≥ 45 min und ≤ 1,5 h. Noch bevorzugter erfolgt die Inkubation für eine Zeitdauer von ungefähr 1 h.

In einer weiteren bevorzugten Ausführungsform erfolgt die Inkubation unter leichter kontinuierlicher oder diskontinuierlicher Durchmischung des Ansatzes, beispielsweise durch Schwenken, Schütteln, Rühren und dergleichen. Noch bevorzugter erfolgt die Durchmischung alle 5, 10, 15, 30, oder 45 min, bevorzugter alle 15 min der Inkubationszeit.

In einer bevorzugten Ausführungsform der Erfindung schließt sich an den Inkubationsschritt zumindest ein Waschschritt, noch bevorzugter ≥ 2, ≥ 3, ≥ 4 oder ≥ 5 Waschschritte an. Dies hat den Vorteil, dass unspezifische Antigene von den Beads entfernt werden und somit die Spezifität und/oder Sensitivität des Verfahrens erhöht wird.

In einer weiteren bevorzugten Ausführungsform erfolgt der zumindest eine Waschschritt auf einem magnetischen Separator, d.h. in der Nähe eines Magneten, der die paramagnetischen Beads zurückhält. Dies hat den Vorteil, dass während des zumindest einen Waschschritts keines der Beads verloren geht.

In einer besonders bevorzugten Ausführungsform wird für zumindest einen des zumindest einen Waschschritts derselbe Waschpuffer verwendet, wie für die Kontaktierung der Beads zu Beginn des erfindungsgemäßen Verfahrens. Am bevorzugtesten wird für jeden der Waschschritte der erfindungsgemäße und identische Waschpuffer wie für die Kontaktierung der Beads verwendet. Es hat sich gezeigt, dass der erfindungsgemäße Waschpuffer besonders für das Waschen der inkubierten Beads geeignet ist und zu einer erhöhten Sensitivität und Spezifität des erfindungsgemäßen Detektionsverfahrens führt. Ein weiterer Vorteil ist, dass nur ein einziger Waschpuffer für das erfindungsgemäße Verfahren bereitgestellt zu werden braucht, was zu einer Einsparung von Zeit und Kosten, sowie Platz in dem erfindungsgemäßen Kit führt.

In einem weiteren Schritt des erfindungsgenmäßen Verfahrens werden die magnetischen Beads mittels eines magnetischen Separators isoliert. Hierbei wird ein Magnet, d.h. der magnetische Separator, in die Nähe der paramagnetischen Beads gebracht, der diese zurückhält und der Überstand der Suspension wird anschließend verworfen. Wenn sich, zum Beispiel die Beads mit der Probe für die Inkubation in einem Gefäß befunden haben, so wird der magnetische Separator von außen an das Gefäß herangebracht, wodurch sich die Beads an die Gefäßinnenwand anlagern und dort festgehalten werden. Danach kann die in dem Gefäß enthaltenen Flüssigkeit, d.h. im Wesentlichen der Waschpuffer und die Probe, ausgegossen werden, wobei die Beads im Gefäß verbleiben und somit isoliert wurden. Falls der zuvor beschriebene Waschschritt nach der Inkubation durchgeführt wurde und ein solcher Separator verwendet wurde, so liegen die Beads bereits z.B. an die Gefäßwand angelagert vor und lediglich der Waschpuffer muss für die Isolierung verworfen werden.

In einem weiteren Schritt des erfindungsgenmäßen Verfahrens wird eine Detektion der mittels der Antikörper an die isolierten Beads gebundenen Antigene durchgeführt. Eine solche Detektion wird vorteilhaft eine signifikant verbesserte Spezifität und/oder Sensitivität, d.h. letztlich eine verbesserte Nachweisgrenze aufweisen, wodurch neben einer höheren Genauigkeit und Verlässlichkeit der Detektion auch ein Zeitgewinn, d.h. eine schneller durchzuführende Detektion ermöglicht wird. Dies wird dadurch ermöglicht, dass durch den verwendeten erfindungsgemäßen Waschpuffer eine verbesserte, d.h. spezifischer, Isolierung der Antigene mittels der Beads erreicht wird.

Die Detektion der an die isolierten Beads gebundenen Antigene kann mittels eines jeden dem Fachmann bekannten Verfahren durchgeführt werden. In bevorzugten Ausführungsformen erfolgt diese Detektion immunologisch und/oder mittels eines Nukleinsäureamplifikationsverfahrens. Ein Nukleinsäureamplifikationsverfahren setzt voraus, dass das Antigen eine Nukleinsäure umfasst, dies ist beispielsweise bei Mikroorganismen wie Bakterien, Viren, Protozoen und oder Pilzen der Fall.

Im Folgenden soll auf zwei spezifische der bevorzugten Detektionsverfahren des immunologischen bzw. des Nukleinsäureamplifikationsverfahrens näher eingegangen werden.

Ein bevorzugtes immunologisches Detektionsverfahren gemäß der Erfindung ist der enzymelinked immunosorbent assay (ELISA), noch bevorzugter der Sandwich-ELISA. Der ELISA ist ein dem Fachmann wohlbekanntes Verfahren, um gezielt bestimmte Moleküle, d.h. Antigene, nachweisen zu können. Dabei nutzt man die Mechanismen des Immunsystems: Wird eine Substanz vom Immunsystem als fremd erkannt, bildet es Antikörper, die an das fremde Molekül andocken und es so markieren. Diese so genannte Antikörper-Antigen-Interaktion wird für den ELISA-Test genutzt. Soll ein bestimmtes Antigen nachgewiesen werden, müssen die dazu passenden Antikörper bekannt sein und zuvor mit verschiedenen gentechnischen oder zellbiologischen Verfahren hergestellt worden sein. Ist dann in einer Probe das gesuchte Molekül vorhanden, so wird es von einem - zuvor auf ein Trägermedium aufgebrachten - Antikörper gebunden und somit aus der Probe heraus isoliert ("gefangen"). Diesem Trägermedium mit den daran gebundenen Antikörpern entsprechen die Antikörper-beschichteten Beads der vorliegenden Erfindung.

Beim Sandwich-ELISA wird an die so immobilisierten Antigene ein zweiter Antikörper gebunden, der Detektiert werden kann, bzw. für die Erzeugung eines detektierbaren Signals verwendet wird. Die bedeutet, dass bei der immunologischen Detektion gemäß der vorliegenden Erfindung ein zweiter, d.h. sogenannter sekundärer Antikörper für das Antigen verwendet wird.

Dem Fachmann sind verschiede Möglichkeiten bekannt, mittels dieses zweiten Antikörpers ein detektierbares Signal zu erzeugen. In einer bevorzugten Ausführungsform ist der zweite Antikörper an ein Enzym gekoppelt, ein sogenanntes Konjugat, mit dessen Hilfe das detektierbare Signal erzeugt wird. Dies kann in einer weiteren bevorzugten Ausführungsform durch das Umsetzen eines Substrats und einer damit erzeugter Lichtabstrahlung und/oder Farberzeugung geschehen. In einer besonders bevorzugten Ausführungsform ist der sekundäre Antikörper an das Enzym "horseradish peroxidase" (HRP) gekoppelt. Eine von diesem Enzym katalysiert Reaktion, bei der 3,3',5,5'-Tetramethylbenzidin (TMB) umgesetzt wird, führt dann zu einem sichtbaren Farbniederschlag, der anschließend mit speziellen Analysegeräten ausgewertet werden kann. Konjugierte Antikörper können beispielsweise von oben genannten Herstellern bezogen werden.

In einer bevorzugten Ausführungsform der Erfindung erfolgt die Detektion der mittels der Antikörper an die Beads gebundenen Antigene daher immunologisch über einen Sandwich-ELISA.

Hierzu umfasst das erfindungsgemäße Verfahren weiterhin den Schritt der Aufnahme mittels des magnetischen Separators der isolierten Beads in einer Konjugat-Lösung gefolgt von einer Inkubation der Beads in dieser Konjugat-Lösung.

In einer bevorzugten Ausführungsform enthält die Konjugat-Lösung einen sekundären, für das Antigen spezifischen Antikörper, der an ein Enzym konjugiert vorliegt. Noch bevorzugter ist das Enzym in der Lage eine Licht- oder Farbreaktion zu katalysieren. Am bevorzugtesten handelt es sich bei dem Enzym um HRP. In einer weiteren Ausführungsform umfasst der Konjugat-Puffer PBS, in dem sekundäre der Antikörper verdünnt vorliegt, bevorzugt liegt der sekundäre Antikörper in einer Konzentration von 1:500, 1:750, 1:1000, oder 1:1500 vor.

Die Inkubation erfolgt durch Inkontaktbringen der isolierten Beads mit der Konjugat-Lösung unter geeigneten Bedingungen, die eine Komplexierung des konjugierten, sekundären Antikörpers mit seinem Antigen, welches auf den Antikörpern der Beads immobilisiert vorliegt, erlauben. In bevorzugten Ausführungsformen wird die Inkubation bei Raumtemperatur oder bei einer Temperatur zwischen ≥ 20° C und ≤ 40° C, ≥ 30° C und ≤ 40° C und noch bevorzugter bei etwa 37° C durchgeführt.

In einer weiteren bevorzugten Ausführungsform erfolgt die Inkubation für eine Zeitdauer von ≥ 1 min und ≤ 3 h, ≥ 10 min und ≤ 2 h, ≥ 20 min und ≤ 2 h, ≥ 45 min und ≤ 1,5 h. Noch bevorzugter erfolgt die Inkubation für eine Zeitdauer von ungefähr 1 h.

In einer weiteren bevorzugten Ausführungsform erfolgt die Inkubation unter leichter kontinuierlicher oder diskontinuierlicher Durchmischung des Ansatzes, beispielsweise durch Schwenken, Schütteln, Rühren und dergleichen. Noch bevorzugter erfolgt die Durchmischung alle 5, 10, 15, 30, oder 45 min, bevorzugter alle 15 min der Inkubationszeit.

In einer bevorzugten Ausführungsform der Erfindung schließt sich an den Inkubationsschritt zumindest ein Waschschritt, noch bevorzugter ≥ 2, ≥ 3, ≥ 4 oder ≥ 5 Waschschritte an. Dies hat den Vorteil, dass unspezifische Antigene von den Beads entfernt werden und somit die Spezifität und/oder Sensitivität des Verfahrens erhöht wird. Für den Waschschritt wird vorzugsweise PBS verwendet.

In einer weiteren bevorzugten Ausführungsform erfolgt der zumindest eine Waschschritt auf einem magnetischen Separator, d.h. in der Nähe eines Magneten, der die paramagnetischen Beads zurückhält. Dies hat den Vorteil, dass während des zumindest einen Waschschritts keines der Beads verloren geht.

In einer weiteren Ausführungsform umfasst das erfindungsgemäße Verfahren weiterhin das Kontaktieren der gewaschenen und mittels des magnetischen Separators isolierten Beads mit einer HRP-Lösung und einer TMB-Substratlösung. In einer bevorzugten Ausführungsform umfasst die HRP-Lösung Kaliumcitrat, noch bevorzugter in einer Konzentration von 30 mM, in Aqua dest. und wurde auf einen pH-Wert von ungefähr 4,1 eingestellt. Hierzu wird vorteilhaft KHO verwendet. In einer weiteren bevorzugten Ausführungsform umfasst die TMB-Substratlösung neben TMB, vorzugsweise in einer Konzentration von 10 mM, Aceton, Ethanol und Wasserstoffperoxid.

Nach Durchmischung des Ansatzes aus isolierten Beads, HRP-Lösung und TMB-Substratlösung erfolgt erfindungsgemäß ein weiterer Inkubationsschritt. In bevorzugten Ausführungsformen wird die Inkubation bei einer Temperatur zwischen ≥ 20° C und ≤ 40° C, ≥ 30° C und ≤ 40° C, oder noch bevorzugter bei Raumtemperatur durchgeführt.

In einer weiteren bevorzugten Ausführungsform erfolgt die Inkubation für eine Zeitdauer von ≥ 1 min und ≤ 2 h, ≥ 10 min und ≤ 1.5 h, ≥ 20 min und ≤ 1 h, ≥ 30 min und ≤ 1 h. Noch bevorzugter erfolgt die Inkubation für eine Zeitdauer von ungefähr 45 min.

Nach erfolgter Inkubation erfolgt ein Abstoppen der Reaktion durch Zugabe einer Stopplösung, vorzugsweise umfassend 1 M H₂SO₄. In einer bevorzugten Ausführungsform wird der Ansatz danach kurz durchmischt und/oder erneut bei einer Temperatur zwischen ≥ 20° C und ≤ 40° C, ≥ 30° C und ≤ 40° C, oder noch bevorzugter bei Raumtemperatur inkubiert. Die Inkubation erfolgt vorteilhaft für eine Zeitdauer von ≥1 min und ≤ 20 min, ≥ 5 min und ≤ 15 min und noch bevorzugter für eine Zeitdauer von ungefähr 10 min.

In einem weiteren Schritt des erfindungsgemäßen Verfahrens erfolgt dann vorzugsweise eine photometrische bzw. colorimetrische Auswertung mittels eines dem Fachmann hinlänglich bekannten Auswertungsgeräts.

Wie weiter oben beschrieben kann die Detektion in einer anderen Ausführungsform der Erfindung auch mittels eines Nukleinsäureamplifikationsverfahrens erfolgen. Wie ebenfalls erwähnt ist hierfür Voraussetzung, dass das "Antigen", d.h. eine Nukleinsäure umfasst. Im Weiteren wird stets von einem "Antigen" gesprochen, auch wenn dieser Begriff eigentlich aus der Immunologie entliehen ist. Dem Fachmann wird jedoch verständlich sein, dass bestimmte mittels des erfindungsgemäßen Verfahrens detektierbare "Stoffe" sowohl ein Antigen darstellen können, als auch eine Nukleinsäure enthalten können. Hierzu zählen z.B. die Mikroorganismen, Bakterien, Viren und Pilze.

Eine Vielzahl von Nukleinsäureamplifikationsverfahren ist dem Fachmann bekannt. Alle solchen Verfahren beruhen im Wesentlichen auf der enzymatischen Vervielfältigung einer Nukleinsäure des Antigens, wodurch eine anschließende Detektion der vervielfältigten Nukleinsäure möglich gemacht wird. Für eine solche Amplifikation sind neben dem Enzym noch sogenannte "Primer" und Nukleotide erforderlich. Weitere Puffer und Substanzen sind dem Fachmann geläufig.

Der Begriff "Nukleinsäure" wie hier verwendet, bezeichnet eine Ribonukleinsäure, vorzugsweise eine Deoxyribonukleinsäure. Diese kann natürlichen Ursprungs sein, d.h. unter anderem aus Organismen, Gewebe, Zellen oder Bioptat gewonnen worden sein. In einer bevorzugten Ausführungsform ist die Nukleinsäure eine DNA (Deoxyribonukleinsäure), eine cDNA, noch bevorzugter eine genomische DNA. Die Nukleinsäure kann einsträngig, doppelsträngig oder mehsträngig vorliegen.

Der Begriff "Amplifikation" wie hier verwendet bezeichnet die Vermehrung einer Nuklein-säure (Template- oder Matrizen-Nukleinsäure) um zumindest den Faktor 2. Bevorzugte Verfahren zur Amplifikation von Nukleinsäuren sind globale Amplifikationsverfahren, die die ganze umgewandelte Nukleinsäure einer Probe amplifizieren. Hierzu zählen isothermale und nicht-isothermale Whole-Genome-Amplification (WGA) Verfahren. Beispiele für Amplifikationsverfahren sind die RCA (Rolling Circle Amplification) und die MDA (Multiple Displacement Amplification). Weitere lineare und exponentielle für die vorliegende Erfindung geeignete Amplifikationsverfahren sind dem Fachmann geläufig, unter anderem PCR (Polymerasekettenreaktion), LCR (Ligase Chainreaction), NASBA (Nucleic Acid Sequence Based Amplification), SDA (Strand Displacement Amplification), TMA (Transcription Mediated Amplification), 3SR (Self-sustained Sequence Replication), LAMP (Loop-mediated Amplifikation), HDA (Helicase dependent Amplification), RPA (Recombinase-Polymerase-Amplification).

Ein im Sinne der vorliegenden Erfindung besonders bevorzugtes Nukleinsäureamplifikationsverfahren ist die PCR. Die PCR ist ein Verfahren, mit dem in einer Kettenreaktion kleinste Mengen eines DNA-Abschnitts vervielfältigt werden können (Amplifikation). Die PCR wird heute sehr oft eingesetzt, wenn anhand bestimmter DNA-Sequenzen Nachweise geführt werden sollen, so etwa in der Kriminalistik/Forensik oder beim Vaterschaftstest, in der Mikrobiologie zur Detektion von lebenden und toten Bakterien (Gesamtzellzahl), in der medizinischen Diagnostik, wenn im Blut oder anderen Körperflüssigkeiten Viren-DNA nachzuweisen ist, in der Evolutionsbiologie, um Verwandtschaftsbeziehungen und Abstammungslinien zu verfolgen und/oder in der Lebensmittelanalytik, um das Vorhandensein von Bestandteilen aus genetisch veränderter Organismen (GVO) nachzuweisen.

Um einen PCR-Nachweis führen zu können, müssen zwei kurze DNA-Stücke (Primer) vorhanden ein, die zu dem gesuchten DNA-Strang passen. Die von ihnen gestartete Kettenreaktion durchläuft mehrere Zyklen, in denen die DNA-Menge mittels einer Polymerase jeweils verdoppelt wird.

Der Begriff "Primer" wie hier verwendet, bezeichnet ein Molekül, das als Startstelle für ein Enzym mit Nukleinsäure-Polymerase- oder Nukleinsäure-Ligase-Aktivität dient. Ein Primer kann ein Protein, eine Nukleinsäure oder ein anderes Molekül sein, das sich dem Fachmann als Polymerase- oder Ligase-Startstelle geeignet erweist. Dieses Molekül kann durch intermolekulare aber auch durch intramolekulare Wechselwirkung als Startstelle dienen. Bevorzugt sind die Primer Nukleinsäure-Primer. Diese können über ihre gesamte Länge mit der Template-Nukleinsäure hybridisieren oder teilweise Misspaarungen aufweisen und weisen in der Regel eine Länge von 4 bis 100 Nukleotiden (nt) auf. Die Länge der Primer liegt bevorzugt zwischen 5 und 50 nt, und ganz besonders bevorzugt zwischen 6 und 25 nt.

Der Begriff "Primersatz" wie hier verwendet, bezeichnet eine Schar von Primern, die zur Durchführung einer Amplifikation benötigt wird und besteht aus zumindest einem Primer. Für lineare Amplifikationen besteht ein Primersatz zumindest aus einem Primer. Für exponentielle Amplifikationen besteht ein Primersatz ebenfalls zumindest aus einem Primer, falls dieser an zumindest zwei unterschiedlichen Positionen der Template-Nukleinsäure hybridisiert, wo-durch der zwischen den Hybridisierungspositionen liegende Nukleinsäureabschnitt exponentiell amplifiziert wird. Bevorzugt besteht der Primersatz bei exponentiellen Amplifikationen jedoch zumindest aus zwei Primern, die an zwei unterschiedlichen Positionen der Template-Nukleinsäure hybridisieren; auch hier wird der zwischen den Hybridisierungspositionen liegende Nukleinsäureabschnitt exponentiell amplifiziert.

Der Begriff "Polymerase" wie hier verwendet, bezeichnet ein Enzym, welches die Bildung von Phosphodiester-Bindungen zwischen einzelnen Nukleotiden innerhalb eines Nukleinsäurestrangs katalysiert (z.B. DNA- und RNA-Polymerasen). Besonders bevorzugt sind für den Einsatz bei dem erfindungsgemäßen Verfahren zur Amplifikation Polymerasen, die für Amplifikationsreaktion geeignet sind, insbesondere alle DNA-Polymerasen. Die Polymerasen las-sen sich unterscheiden in hitzelabile- oder hitzestabile Enzyme. Nicht alle Polymerasen sind für unterschiedlich Amplifikationsverfahren gleichgut geeignet. So eignen sich Polymerasen mit Strand-Displacement Aktivität für isothermale Amplifikationsverfahren, während hitze-stabile Polymerasen für nicht-isothermale Amplifikationsverfahren bevorzugt sind. Bevorzugte Polymerasen schließen Polymerasen mit Enzym-Nummer EC 2.7.7.7, Taq-Polymerase, Polymerasen mit Proof-Reading Aktivität, Polymerasen mit Strand-Displacemenet Aktivität, mutierte Polymerase, wie auch Polymerasen mit akzesorischen Faktoren (z.B. Helicase, Ein-zelstrang-Bindungsproteine, Rekombinationsproteine) und Holoenzyme, die einen funktionellen DNA-Polymerase Komplex bilden, ein.

In einer bevorzugten Ausführungsform ist die Polymerase eine DNA-Polymerase, noch bevorzugter eine Taq-Polymerase, eine DNA-Polymerase mit Proof-Reading Aktivität, oder eine Polymerase mit Strand-Displacement Aktivität. Eine Strand-Displacement Aktivität ist eine Eigenschaft einer Polymerase, durch welche während der Polymerase-Reaktion ein Abschälen eines "alten" Stranges ("Strand-Displacement") einer doppelsträngigen Nukleinsäure von dem anderen "alten" Strang bewirkt wird. Zu den Strand-Displacement-Polymerasen gehören alle Polymerasen, die ein Strand-Displacement durchführen können. Hierzu zählen Enzyme, wie z.B. Phi29-DNA-Polymerase, Klenow exominus DNA-Polymerase, Vent DNA Polymerase, Deep VentTM DNA Polymerase, Bst DNA Polymerase, 9oNm™ DNA Polymerase und Bca DNA Polymerase.

Die Strand-Displacement-Polymerasen können auch in mutierter Form vorliegen, z.B. als sogenannte exominus-Varianten (d.h. ohne Exonuklease Aktivität). Hierzu zählen Polymerase wie die Phi29-DNA-Polymerase, Klenow exominus DNA-Polymerase. Des Weiteren können der Polymerase weitere akzessorische Faktoren zugegeben werden, die eine Amplifikation der mittels Bisulfitierung umgewandelte Nukleinsäure verbessern oder ermöglichen. Zu diesen Faktoren gehören z.B. Helicasen, Einzelstrang-DNA-Bindungsproteine (z.B. SSB, T4gp32), Rekombinationsproteine (z.B. recA, Mut Proteine).

Der Begriff "Ligase" wie hier verwendet, bezeichnet eine Ligase, die zwei Nukleinsäure-Stränge über die Herstellung einer Phosphor-Esterbindung verknüpft (ligiert). Hierzu zählen DNA- sowie RNA-Ligasen. Somit bezeichnet "Ligase" ein Enzym mit der Enzym-Nummer EC 6.5.1.1, EC 6.5.1.2 bzw. EC 6.5.1.3.

In einer bevorzugten Ausführungsform der Erfindung erfolgt die Detektion der mittels der Antikörper an die Beads gebundenen Antigene daher mittels eines Nukleinsäureamplifikationsverfahrens. In einer besonders bevorzugten Ausführungsform ist das Nukleinsäureamplifikationsverfahren eine PCR.

Hierzu umfasst das erfindungsgemäße Verfahren weiterhin den Schritt der Aufnahme zumindest eines Teils der mittels des magnetischen Separators isolierten Beads in einen Nukleinsäureamplifikationsansatz.

In einer bevorzugten Ausführungsform umfasst der Nukleinsäureamplifikationsansatz Tris-HCl, vorzugsweise bei einem pH von 8,3, KCl, MgCl₂, einen Nukleotidmix der vier Nukleotide dATP, dTTP, dGTP und dCTP, sowie ein Amplifikationsenzym. Noch bevorzugter ist der Nukleinsäureamplifikationsansatz ein PCR-Reaktionsansetz und umfasst 10 mM Tris-HCl, vorzugsweise bei einem pH von 8,3, 50 mM KCl, 1,5 mM MgCl₂, einen Nukleotidmix der vier Nukleotide dATP, dTTP, dGTP und dCTP mit einer Konzentration von jeweile 200 µM, sowie Taq DNA-Polymerse, vorzugsweise in einer Konzentration von 0,02 U.

Der Nukleinsäureamplifikationsansatz umfasst weiterhin zumindest einen für das Antigen spezifischen Primer, bevorzugt ein für das Antigen spezifisches Primerpaar. Ein Primer oder Primerpaar wird dann spezifisch für ein bestimmtes Antigen angesehen, wenn es unter Hochsalzbedingungen mit der Nukleinsäure des Antigens hybridisiert. Vorzugsweise weist ein spezifischer Primer oder ein spezifisches Primerpaar eine vollständige Sequenzidentität zu der Nukleinsäure des zu detektierenden Antigens auf. In einer weiteren bevorzugten Ausführungsform werden pro Reaktionsansetz 100 pMol jeden Primers eingesetzt.

In besonders bevorzugten Ausführungsformen ist das zu detektierende Antigen Legionella pneumophila und der Primer und/oder das Primerpaar ist spezifisch für das ompS-Gen von Legionella pneumophila. Das Markergen ompS ist ein hochkonservatives Gen, das für ein Membranprotein kodiert. Die Bezeichnung "hochkonservativ" bedeutet, dass dieses Markergen für die Detektion unterschiedlicher Serogruppen (Legionella-Serogruppe 1-12) geeignet ist. Dieses Markergen wurde bisher noch nie für einen schnellen und sensitiven Test verwendet. In der hier vorliegenden Erfindung wird das ompS-Gen zum ersten Mal zur Detektion von Legionella pneumophila benutzt. In einer besonders bevorzugten Ausführungsform wird das Primerpaar mit der folgenden Sequenz eingesetzt: ompS_Leg_forward 5' -GCG GCT GTA TTT GCT CTG GGA A- 3' (SEQ ID NO: 1) und ompS_Leg_reverse 5' -TAA GCC TAT GTA GGG GCC AGA TGC- 3' (SEQ ID NO: 2).

In weiteren besonders bevorzugten Ausführungsformen ist das zu detektierende Antigen ein Adenovirus und der Primer und/oder das Primerpaar ist spezifisch für das adenovirale Hexon-Gen. Das Hexon-Markergen kodiert ebenfalls für ein Oberflächenprotein und wird im Gegensatz zu dem ompS-Gen routinemäßig in der Diagnostik eingesetzt. Auf Grund einer sehr hohen Mutationsrate ist die Sequenz des Gens einer ständigen Veränderung unterworfen. Hochkonservative Bereiche über mehrere Serogruppen sind daher sehr selten. Durch intensive Recherche ist es jedoch gelungen zwei solcher bisher unbekannter Bereiche zu identifizieren. In diesen Bereichen wurden hochspezifische Hexon-Primer designt, die eine schnelle und sehr sensitive Detektion ermöglichen. Bisher wurden diese Bereiche noch nie für eine Detektion verwendet und die Sequenz der Detektions-Primer wird erstmals in dieser Erfindung offenbart. In einer besonders bevorzugten Ausführungsform wird das Primerpaar mit der folgenden Sequenz eingesetzt:
hexon_AdV_forward5' -GAA ATG ACA CCA ACG ACC AG- 3' (SEQ ID NO: 3) und
hexon_AdV_reverse 5' -GGG AAC ATT AGC GGG GTA AG- 3' (SEQ ID NO: 4).

In einem weiteren Schritt des erfindungsgemäßen Verfahrens erfolgt nun zumindest eine Amplifikationsrunde. Die Art und Weise, wie eine solche Amplifikationsrunde durchgeführt wird hängt sowohl vom verwendeten Amplifikationsenzym als auch vom angewandten Amplifikationsverfahren ab.

Bei dem bevorzugten PCR Amplifikationsverfahren erfolgt die Amplifikation in mehreren Zyklen, vorzugsweise ≥ 20, ≥ 25, ≥ 30, oder ≥ 35 Zyklen, mit jeweils drei Reaktionsschritten. Im ersten Reaktionsschritt wird der DNA-Doppelstrang durch Erhitzen in Einzelstränge aufgeschmolzen. Dies erfolgt bevorzugt bei ungefähr 95° C. In einem zweiten Schritt wird die Temperatur zum Annealing der Primer abgesenkt. Die jeweilige optimale Annealingtemperatur hängt von der Länge und der Sequenz der verwendeten Primer ab und kann vom Fachmann ohne Probleme berechnet werden. Im dritten Reaktionsschritt werden in Gegenwart der Nukleotide (dNTPs) von den Primern ausgehend Doppelstränge synthetisiert ("Elongation"), wozu die Temperatur auf die optimale Reaktionstemperatur der Polymerase, im Falle der Taq-Polymerase 72° C erhöht wird. Die Elongationsszeit hängt von der Länge des zu amplifizierenden Fragments ab und kann ebenfalls vom Fachmann berechnet werden. Im Allgemeinen wird pro 1kb zu amplifizierender Nukleinsäure eine Elongationszeit von 1min angenommen. Die entstandenen DNA-Doppelstränge dienten ihrerseits im nächsten Zyklus als Matrize, was eine exponentielle Amplifikation des DNA-Fragments zur Folge hat.

Beispielhaft kann die PCR-Amplifikation unter folgenden Bedingungen erfolgen: 1) Denaturierung: 95° C für 5 min, 2) Denaturierung: 95° C für 20 s, 3) Annealing der Primer: für Legionella pneumophila bei 58° C, für Adenovirus bei 54° C für 20 s, 4) Synthese, d.h. Elongation bei 72° C für eine Minute. Die Schritte 2) bis 4) werden für 30-35 Zyklen wiederholt, gefolgt von einem abschließenden Elongationsschritt bei 72° C für 5 min.

Nach erfolgter Amplifikation werden Aliquots des Reaktionsansatzes bevorzugt mit Hilfe der Gelektrophorese aufgetrennt und die enthaltenen Nukleinsäuren durch Färbung sichtbar gemacht. In noch bevorzugteren Ausführungsformen ist die Gelelektrophorese eine Polyacrylamid-oder Agarosegelelektrophorese und/oder die Anfärbung erfolgt mittels Ethidiumbromid und UV-Bestrahlung.

In einem weiteren Aspekt ist die Anmeldung auf einen Waschpuffer gerichtet, der wie oben ausgeführt zusammengesetzt sein kann. In einer bevorzugten Ausführungsform ist der Waschpuffer ein PBS-Waschpuffer umfassend zumindest 8 % und bevorzugt 10 % BSA sowie zumindest ein Detergenz.

In einer bevorzugten Ausführungsform ist das Detergenz Tween 20 und/oder Triton-X und liegt noch bevorzugter in einer Konzentration von 0,01 Vol-% vor.

In einer besonders bevorzugten Ausführungsform umfasst der Waschpuffer weiterhin Kaliumchlorid 2,7 mM, Kaliumdihydrogenphosphat 1,5 mM, Dinatriumhydrogenphosphat 8,1 mM und/oder Natriumchlorid 136,9 mM.

Ebenfalls besonders bevorzugt wird der Waschpuffer mit Aqua dest. aufgefüllt und mittels HCl oder NaOH auf einen pH-Wert von 7,4 eingestellt.

Ein einem weiteren Aspekt umfasst die vorliegende Erfindung die Verwendung des Waschpuffers in einem erfindungsgemäßen Verfahren.

In einem weiteren Aspekt ist die Anmeldung auf einen Primer gerichtet, der wie oben ausgeführt ausgewählt ist aus der Gruppe bestehende aus einem SEQ ID NO: 1, 2, 3 und 4. Desweiteren werden auch Primer von der Erfindung umfasst, die eine in den SEQ ID NO: 1, 2, 3 oder 4 wiedergegebene Sequenz umfassen und/oder ein Homolog zu einer solchen Sequenz darstellen. Bevorzugt weist ein solches Homolog eine Nukleinsäureidentität von ≥ 80 %, ≥ 85 %, ≥ 90 %, ≥ 91%, ≥ 92%, ≥ 93%, ≥ 94%, ≥ 95%, ≥ 96 %, ≥ 97 %, ≥ 98 %, oder ≥ 99 % auf.

Ein einem weiteren Aspekt umfasst die vorliegende Anmeldung die Verwendung der erfindungsgemäßen Primer in einem der erfindungsgemäßen Verfahren.

In einem weiteren Aspekt ist die Erfindung auf ein Kit nach Anspruch 13 ausgerichtet, welches zumindest eine Art der erfindungsgemäßen Beads, die mit zumindest einem Antikörper für zumindest ein Antigen beschichtet sind, sowie den erfindungsgemäßen Waschpuffer umfassend zumindest 8 % und bevorzugt 10 % BSA umfasst.

In einer bevorzugten Ausführungsform umfasst das Kit weiterhin eine, zwei, drei, vier oder fünf der Komponenten gemäß der vorliegenden Erfindung ausgewählt aus der Gruppe bestehend aus: Positivkontrolle; Negativkontrolle; Reaktionsgefäße; magnetischer Separator und Bedienungsanleitung.

In einer weiteren bevorzugten Ausführungsform umfasst das Kit weiterhin eine, zwei, drei oder vier der Komponenten der vorliegenden Erfindung ausgewählt aus der Gruppe bestehend aus: sekundärer Antikörper (Konjugat); HRP-puffer; TMB-Substratlösung und Stopplösung.

In einer weiteren bevorzugten Ausführungsform umfasst das Kit weiterhin eine, zwei oder drei der Komponenten der vorliegenden Erfindung ausgewählt aus der Gruppe bestehend aus: Primer spezifisch für ein Antigen, vorzugsweise ein PCR-Primerpaar; Nukleinsäureamplifikationspuffer, vorzugsweise PCR-Reaktionspuffer und vorzugsweise separat vom Nukleotid-Mix und dem Nukleinsäure-amplifizierenden Enzym, vorzugsweise DNA-Polymerase; Entionisiertes H₂O.

In einem weiteren Aspekt umfasst die Anmeldung eine Vorrichtung zum Ausführen des Verfahrens der vorliegenden Erfindung. Aufgrund der oben gemachten Ausführungen werden sich die Komponenten der Vorrichtung dem Fachmann leicht erschließen. Insbesondere umfasst eine solche Vorrichtung Mittel zur Probenentnahme, ein photometrisches oder colorimetrisches Auswertegerät und/oder einen Thermocylcer sowie eine Gelelektrophoreseapparatur.

Das Mittel zur Probenentnahme kann jedes geeignete Mittel sein, um der Vorrichtung eine geeignete Probe zuzuführen. Beispielhafte Mittel für die Probenentnahme schließen Spritzen, Pipetten, Pinzetten und Ansaugeinrichtungen ein, ohne darauf beschränkt zu sein.

Das photometrische oder colorimetrische Auswertegerät kann beispielsweise jegliche dem Fachmann bekannte Vorrichtung zum Auslesen von ELISA-Platten sein. Gleichermaßen können der Thermocylcer sowie die Gelelektrophoreseapparatur ebenfalls jegliche geeignete und dem Fachmann bekannte Vorrichtung zur Durchführung einer Nukleinsäureamplifikation bzw. einer Gelektrophorese sein.

In einer bevorzugten Ausführungsform ist die Vorrichtung eine mobile Vorrichtung. Noch bevorzugter ist die Vorrichtung tragbar und/oder auf Rollen, Rädern oder ähnlichem bewegbar. Dies hat den Vorteil, dass das erfindungsgemäße Verfahren leicht und schnell an nahezu jedem Ort durchgeführt werden kann, ohne das die Proben nach der Entnahme zur Analyse zunächst in ein Labor verschickt werden müssen.

In einer weiteren bevorzugten Ausführungsform ist die Vorrichtung ausgelegt das erfindungsgemäße Verfahren halb- oder vollautomatisch auszuführen. Bevorzugt kann bei Detektion eines bestimmten Antigens durch die Vorrichtung ein Alarm ausgelöst werden. Dies ist besonders für Sicherheitsanwendungen, wie z.B. am Flughafen, von Vorteil, bei denen es auf eine kontinuierliche und automatische Überwachung auf bestimmte Substanzen, wie z.B. biologische Waffen oder Drogen, ankommt.

In einem weiteren Aspekt ist die Anmeldung auf die Verwendung des/der Verfahrens, Waschpuffers, Primer, Kits und Vorrichtung für folgende Anwendungen ausgerichtet:

Schneller und empfindlicher Nachweis von Mikroorganismen und anderen gefährlichen Substanzen, insbesondere biologische Toxine. Schneller Nachweis von Krankheitserregern aus Körperflüssigkeiten, insbesondere Blut, Speichel, Serum, Tränenflüssigkeit und Urin im Bereich der medizinischen Diagnostik. Sicherer Nachweis von biologischen Gefahrstoffen zur Erkennung und Verhinderung von biologischen Terroranschlägen. Verhinderung von Epidemien und Pandemien durch einen schnellen und sichern Nachweis von Infektionskrankheiten (z.B. SARS) am Flughafen oder andern Infrastrukturen. Schneller Nachweis von Mikroorganismen und gefährlichen Substanzen in der Lebensmittelüberwachung. Schutz von kritischen Infrastrukturen (Gericht, Behörden, Bundeseinrichtungen usw.) Schneller Nachweis von Mikroorganismen bei Weltraummissionen und zum aufspüren extraterrestrischen Lebens.

Legionellen treten überwiegend in Warmwassersystemen und lüftungstechnischen Anlagen auf. Die Gefahr des Einatmens infektiöser Aerosole ist in Duschen und Whirlpools besonders hoch, insbesondere bei geringer und unregelmäßiger Wasserentnahme. Lüftungstechnische Anlagen können durch Emission infektiöser Aerosole in die Umgebungsluft eine nicht zu unterschätzende Infektionsquelle darstellen. Daher ist der erfindungsgemäße Nachweis von Legionella in allen stehenden und fließenden Wasserbehältnissen und/oderLüftungsanlagen zum Schutz folgender Infrastruktur (Gebäude und Systeme) wichtig: Schiffe der Marine, U-Boote, Kasernen, Krankenhäuser und/oder Feldlazarette (z.B. bei Auslandseinsätzen), Passagierflugzeuge (z.B. A380 mit Dusche), Alten- und Pflegeheime, Hotels, Herbergen, Arzt- und Zahnarztpraxen, Wasserversorger aller Art, Campingplätze, Freizeitanlagen insbesondere solche mit Whirlpools, Schwimmbädern, Springbrunnen, Mehrfamilienhäuser, Schulen, Kindergärten, Passierschiffen, Justizvollzugsanstalten, Schwimm- und Sportanlagen, Versuchstierställe, Gewerbliche Immobilien (Produktion), Ein- und Zweifamilienhäuser (mit und ohne Klimaanlage), Mehrfamilienhäuser (mit und ohne Klimaanlage).

Die weltweit verbreiteten Adenoviren sind äußerst resistent gegen Umwelteinflüsse und bei Zimmertemperatur wochenlang stabil. Deswegen können in Gemeinschaftseinrichtungen oft kleine adenovirale Epidemien auftreten. Besonders betroffen sind dabei z.B. Schwimmbäder oder Kasernen. Für die Eindämmung solcher Epidemien ist eine erfindungsgemäße schnelle und sensitive Detektion sehr wichtig.

### BESCHREIBUNG DER ZEICHNUNGEN

- Fig. 1: zeigt das Ergebnis eines Blockierungsexperiments unspezifischer Bindungsstellen der Beads.
- Fig. 2: zeigt die erfindungsgemäße Detektion von Legionella pneumophila mittels eines immunologischen Verfahrens.
- Fig. 3: zeigt die erfindungsgemäße Detektion von Adenovirus mittels eines immunologischen Verfahrens.
- Fig. 4: zeigt die erfindungsgemäße Detektion von E. coli mittels eines immunologischen Verfahrens.
- Fig. 5: zeigt den erfindungsgemäßen qualitativen und quantitativen Nachweis von Legionella pneumophila mittels eines Nukleinäureamplifikationsverfahrens.

### DETAILLIERTE BESCHREIBUNG DER ERFINDUNG

### BEISPIELE

### Beispiel 1: Herstellung der Antikörper-beschichteten Beads

Für die folgenden Detektionsexperimente von Antigenen mittels des erfindungsgemäßen Verfahrens wurden Streptavidin-Beads und folgende primäre und sekundäre IgG Antikörper, bzw. Antikörperpaare verwendet:

| **Antikörper** | **Organismus** | **Firma** |
|---|---|---|
| Anti *Adenovirus,* Biotin konjugiert | Ziege, polyklonal | Acris Antibodies |
| Anti *Adenovirus,* HRP konjugiert | Ziege, polyklonal | Acris Antibodies |
| | | |
| Anti *E.coli,* Biotin konjugiert | Kaninchen, polyklonal | Novus Biologicals |
| Anti *E.coli,* HRP konjugiert | Kaninchen, polyklonal | Novus Biologicals |
| | | |
| Anti *L.pneumophila* | Kaninchen, polyklonal | Acris Antibodies |
| Anti *L.pneumophila,* Biotin konjugiert | Kaninchen, polyklonal | Acris Antibodies |
| | | |
| verwendete Kontrollantikörper: | | |
| Anti Ziege IgG, HRP konjugiert | Kaninchen, polyklonal | Abcam |
| Anti Kaninchen IgG, HRP konjugiert | Ziege | Novus Biologicals |

Zur Herstellung der Antikörper-beschichteten Beads wurden die Streptavidin-Beads mit jeweils einem der Biotin-Konjugierten Antikörper inkubiert.

### Beispiel 2: Optimierung des Waschpuffers

Ein erfindungswesentlicher Bestandteil des Waschpuffers ist das in einer bestimmten Konzentration vorliegende BSA. Um die optimale Konzentration an BSA für das erfindungsgemäße Verfahren zu ermitteln wurden Testreihen durchgeführt, bei denen ein unspezifisches Binden von Adenoviren an die Beads selbst untersucht wurde. Hierzu wurden die Beads über Nacht mit unterschiedlichen Blockierungsreagenzien (0,1 % BSA; 10 % BSA; 5 % Milchpulver und 0,5 mg/ml Biotin) inkubiert. Parallel wurden Kontrollexperimente durchgeführt, um auszuschließen, dass die eingesetzten Blockierungsreagenzien die Interaktion zwischen Antigen und Antikörper behindern. Die Beads wurden jeweils mit dem Waschpuffer unterschiedlicher BSA Konzentrationen geblockt und nach Inkubation mit den Adenoviren mit demselben Puffer gewaschen wurden, bevor eine Adenoviren-spezifische PCR durchgeführt wurde.

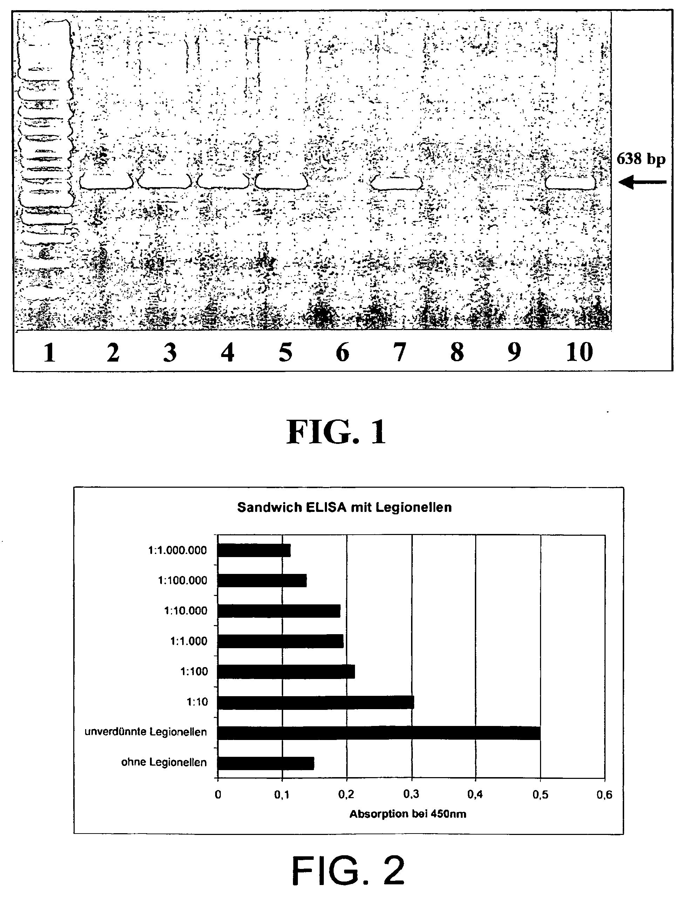

Fig. 1 zeigt das Ergebnis dieses Experiments eines Blockens unspezifischer Bindungsstellen der Beads mit nachgeschalteter Adenoviren-spezifischen PCR. Insbesondere zeigt Spur 1) DNA-Marker und Spuren 2-5 Positivkontrollen, wobei Spur 2) Beads beschichtet mit Adenoviren-spezifischen Antikörpern, 0,1 % BSA im Waschpuffer und Inkubation mit Adenoviren zeigt, Spur 3) Beads beschichtet mit Adenoviren-spezifischen Antikörpern, 10 % BSA im Waschpuffer und Inkubation mit Adenoviren zeigt, Spur 4) Beads beschichtet mit Adenoviren-spezifischen Antikörpern, 5% Milchpulver im Waschpuffer und Inkubation mit Adenoviren zeigt, Spur 5) Beads beschichtet mit Adenoviren-spezifischen Antikörpern, 0,5 mg/ml Biotin im Waschpuffer und Inkubation mit Adenoviren zeigt. Spur 6) ist leer.

Die Ergebnisse des Blockierungstests sind in den Spuren 7-10 zu sehen, wobei Spur 7) unbeschichtete Beads mit 0,1 % BSA im Waschpuffer und Inkubation mit Adenoviren zeigt; Spur 8) Beads mit 10 % BSA im Waschpuffer und Inkubation mit Adenoviren zeigt; Spur 9) Beads mit 5 % Milchpulver im Waschpuffer und Inkubation mit Adenoviren zeigt; Spur 10) Beads mit 0,5 mg/ml Biotin im Waschpuffer und Inkubation mit Adenoviren zeigt.

Als Ergebnis ist deutlich zu erkennen, dass bei ungenügender Blockierung Adenoviren unspezifisch an Beads binden und der entsprechende Genomabschnitt in der anschließenden PCR amplifiziert wird (Spuren 7 und 10). Bei ausreichender Blockierung werden die Adenoviren nicht unspezifisch gebunden und es findet keine Amplifikation statt.

Das Experiment wurde mit Legionellen und vergleichbaren Versuchsergebnissen wiederholt.

Zusammenfassend konnte gezeigt werden, dass bei einer geringeren Konzentration von BSA (z. B. 0,1 %) das Antigen (Legionellen oder Adenoviren) unspezifisch an die magnetischen Beads bindet und es nicht zu der erwünschten erfindungsgemäßen spezifischen Antigen-Antikörper Reaktion kommt.

Es konnte experimentell gezeigt werden, dass ein Blocken (absättigen ungenutzter Bindungsstellen auf den Beads) vor und während des Waschens eine unspezifische Interaktion massiv verhindern kann. Das Blocken der Beads mit 10 % BSA über Nacht lieferte die besten Ergebnisse. Auch ein Blocken der Beads mit 8 % BSA lieferte sehr gute Ergebnisse. Aus diesem Grund wurde dem Waschpuffer (PBS) BSA in einer Bandbreite von 3-12% zugesetzt.

### Beispiel 3: Immunologische Detektion

Das erfindungsgemäße Verfahren wurde mit anschließender immunologischer Detektion durchgeführt.

### Hierzu wurden die oben aufgeführten Antikörperpaare verwendet, sowie folgende Lösungen:

### PBS Waschpuffer:

| | Menge | Endkonzentration |
|---|---|---|
| Kaliumchlorid | 0,2 g | 2,7 mM |
| Kaliumdihydrogenphosphat | 0,2 g | 1,5 mM |
| Dinatriumhydrogenphosphat | 1,15 g | 8,1 mM |
| Natriumchlorid | 8,0 g | 136,9 mM |
| BSA, biotinfrei | 100g | 8% |
| Tween 20 | 0,1 ml | 0,01% |
| Aqua. dest. | ad 1000 ml | |
| pH 7,4 mit Salzsäure oder Natronlauge eingestellt. | | |

### Konjugat-Lösung

Der konjugierte Antikörper wurde in PBS 1:750 verdünnt.

### HRP-Puffer

| | | |
|---|---|---|
| Kaliumcitrat | 6,9 g | 30 mM |
| Aqua. dest. | ad 1000 ml | |
| pH 4,1 mit Kaliumhydroxid einstellen | | |

### TMB-Substratlösung

| | | |
|---|---|---|
| Tetramethylbenzidin | 240 mg | 10 mM |
| Aceton (96 %) | 10 ml | |
| Ethanol (96 %) | 90 ml | |
| Wasserstoffperoxid (30%) | 750 µl | |

### Stopplösung

| | | |
|---|---|---|
| 96 % Schwefelsäure | 6 ml | 1M |
| Aqua. dest. | ad 95 ml | |

Der Ablauf des Verfahrens verlief nach folgendem Schema:
1) Entnahme von 8 µl mit Antikörper beschichteten Beads (7-12 x10⁸ Beads/ml) und Überführung in das Reaktionsgefäß
2) 5 x Waschen der Beads auf dem magnetischer Separator mit je 400 µl PBS
3) Aufnahme in 8 µl PBS
4) Zugabe von 36 µl Probe
5) Inkubation von 1h bei Raumtemperatur, alle 15 min Durchmischung der Probe
6) 5 x Waschen der Beads auf dem magnetischer Separator mit je 400 µl PBS
7) Aufnahme in 200 µl Konjugat-Lösung
8) Inkubation von 1h bei Raumtemperatur, alle 15 min Durchmischung der Probe
9) 5 x Waschen der Beads auf dem magnetischer Separator mit je 400 µl PBS
10) Aufnahme der Beads in 20 µl PBS
11) In ein Reaktionsgefäß 1000 µl HRP-Puffer + 50 µl TMB-Substratlösung pipettieren und Zugabe der 20 µl Beads aus Schritt 10
12) Mischen der Probe
13) Inkubation von 45 min bei Raumtemperatur
14) Abstoppen der Reaktion mit 500 µl Stopplösung
15) Mischen der Probe
16) Inkubation für 10 min bei Raumtemperatur
17) Auswerten des Assays mit dem Auswertungsgerät

Fig. 2 zeigt die Ergebnisse der Detektion von Legionella pneumophila, wobei unterschiedliche Legionellen-Verdünnungen mit dem oben beschriebenen Verfahren detektiert wurden. Das Detektionslimit lag bei einer Verdünnung von 1:10.000, welches einer Gesamtzellzahl von 7416 Legionellen entsprach.

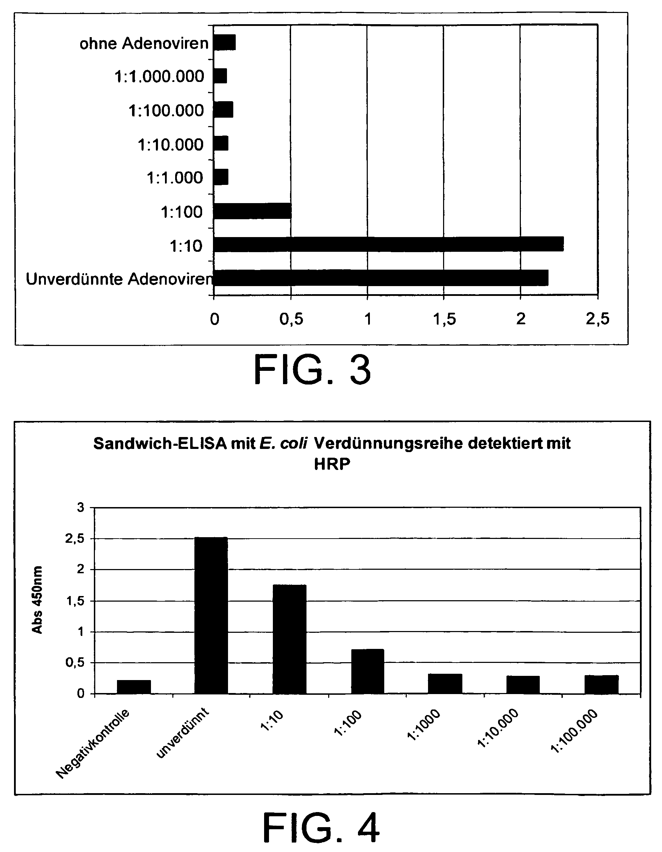

Fig. 3 zeigt die Ergebnisse der Detektion von Adenovirus (Subgruppe C, Serotyp 6), wobei die Adenoviren in Form einer Verdünnungsreihe ausgedünnt und mit den mit spezifischem Antikörper beschichteten Beads inkubiert wurden. Die eigentliche Detektion erfolgte anschließend ebenfalls mit dem Konjugat über die enzymatische Reaktion der "horseradish peroxidase" (HRP). Es konnten spezifisch Adenoviren bis zu einer Verdünnung von 1:100 nachgewiesen werden.

Fig. 4 zeigt die Ergebnisse der Detektion von E. coli., wobei die Bakterien in Zehnerschritten verdünnt und jeweils mit den magnetischen Beads inkubiert wurden. Nach stringentem Waschen wurden die Bakterien ebenfalls colorimetrisch mit TMB als Substrat detektiert. Es konnten E. colis bis zu einer Verdünnung von 1:100 nachgewiesen werden, was einer Gesamtzellzahl von 6000 Bakterien entspricht (nach Kultivierung der Bakterien auf Petrischalen konnte bei der 1:100 Verdünnung eine Zellzahl von 6000 Organismen bestimmt werden).

### Beispiel 4: Detektion über Nukleinsäureamplifikation (PCR)

Neben der immunologischen Detektion von Legionella pneumophila und Adenovirus wurden auch molekularbiologische Tests mit der PCR-Methode entwickelt. Das erfindungsgemäße Verfahren wurde demnach mit anschließender Detektion über eine PCR durchgeführt.

### Hierzu wurden die oben aufgeführten Antikörper für die Herstellung der beschichteten Beads verwendet, sowie folgende Lösungen:

### PBS Waschpuffer:

| | Menge | Endkonzentration |
|---|---|---|
| Kaliumchlorid | 0,2 g | 2,7 mM |
| Kaliumdihydrogenphosphat | 0,2 g | 1,5 mM |
| Dinatriumhydrogenphosphat | 1,15 g | 8,1 mM |
| Natriumchlorid | 8,0g | 136,9 mM |
| BSA, biotinfrei | 100g | 10% |
| Tween 20 | 0,1ml | 0,01% |
| Aqua. dest. | ad 1000 ml | |
| pH 7,4 mit Salzsäure oder Natronlauge eingestellt. | | |

### PCR Reaktionspuffer mit MgCl₂

10 mM Tris-HCl (pH 8.3),
50 mM KCl
1,5 mM MgCl₂

### Nukleotid-Mix

Pro Test wurde eine finale Konzentration von 200µM Nukleotid-Mix eingesetzt. Die Stocklösung betrug 10 mM.

### Taq DNA-Polymerase

Pro Test wurden 0,02 Units/µl Taq DNA-Polymerase verwendet.

Es wurden folgende Primerpaare für die spezifische Detektion von Legionella pneumophila und Adenovirus verwendet:

| **Legionella pneumophila:** | **Primersequenz** |
|---|---|
| ompS_Leg_forward | 5' -GCG GCT GTA TTT GCT CTG GGA A- 3' |
| ompS_Leg_reverse | 5' -TAA GCC TAT GTA GGG GCC AGA TGC- 3' |

| **Adenovirus:** | **Primersequenz** |
|---|---|
| hexon_AdV_forward | 5' -GAA ATG ACA CCA ACG ACC AG- 3' |
| hexon_AdV_reverse | 5' -GGG AAC ATT AGC GGG GTA AG- 3' |

Pro Test wurden jeweils 100 pmol pro Primer eingesetzt.

Diese Primerpaare sind spezifisch für bestimmte Markergene in Legionella und Adenovirus.

Der Nachweis für Legionella pneumophila erfolgte jeweils mit spezifischen Primern für das Gen ompS, das für ein hoch konservatives Membranprotein kodiert. Die Primer dieses Primerpaars liegen an den Positionen 294-315 und 1119-1142 der dem Fachmann bekannten DNA-Sequenz des ompS Proteins von L. pneumophila (Gene-Bank Accession Nummer:M76178.1).

Der Nachweis für Adenovirus erfolgte jeweils mit spezifischen Primern für das Capsid-Gen Hexon. Hier wurde das Primerpaar anhand konservierter Bereiche designt. Die Primer dieses Primerpaars liegen an den Positionen 1937-1956 und 2555-2574 der dem Fachmann bekannten DNA-Sequenz des Hexon Proteins von Adenoviren (Gene-Bank Accession Nummer:AB330087.1).

Zur Amplifikation von DNA-Fragmenten beider Mikroorganismen wurde jeweils ein entsprechendes der oben aufgeführten Primerpaar eingesetzt. Die Amplifikation erfolgte in mehreren Zyklen mit jeweils drei Reaktionsschritten. Im ersten Reaktionsschritt wurde der DNA-Doppelstrang durch Erhitzen auf 95°C in Einzelstränge aufgeschmolzen, an die sich beim schnellen Abkühlen die komplementären Oligonukleotide anlagerten. Im dritten Reaktionsschritt wurden in Gegenwart der Nukleotide (dNTPs) von den Primern ausgehend Doppelstränge synthetisiert, wobei die Temperatur auf 72°C, die optimale Reaktionstemperatur der Polymerase, erhöht wurde.

Unter folgenden Bedingungen erfolgte die Amplifikation:

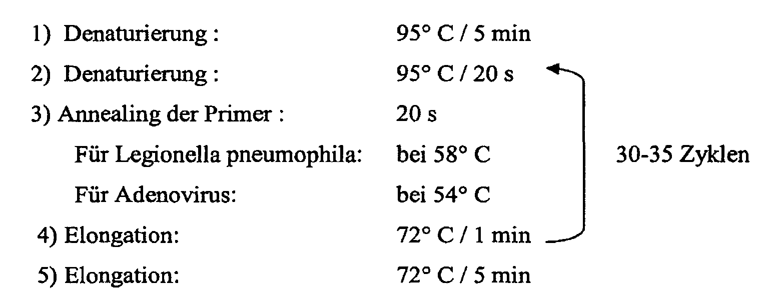

Zur ihrer vollständigen Denaturierung wurde die Matrizen-DNA vor Beginn der Amplifikationsrunde 5 min auf 95°C erhitzt. Anschließend erfolgte in 30 bis 35 Zyklen die Amplifikation des DNA-Fragments. Als Anlagerungstemperatur wurde der Wert des Primers mit der geringeren Schmelztemperatur gewählt. Die Polymerisationszeit wurde nach der Länge des zu amplifizierenden Fragments zwischen 20s und 3min gewählt. Pro 1kb wurde eine Polymerisationsdauer von 1min angenommen. Nach dem letzten Polymerisationsschritt bei 72°C wurde diese Temperatur für weitere 5min beibehalten, um eine vollständige Polymerisation sicherzustellen. Es wurden 50µl-Ansätze in 0,5ml Reaktionsgefäßen vorbereitet.

### Versuchsergebnisse zu Legionella pneumophila

Es wurden Versuche zur qualitativen und quantitativen Detektion mittels Markergen ompS durchgeführt. Für die Detektion von Legionella pneumophila über PCR wurden jeweils 5µl Beads eingesetzt, an die zuvor Legionellen aus einer Verdünnungsreihe gebunden wurden. Der Nachweis erfolgte jeweils mit spezifischen ompS-Primern. Als Negativkontrollen wurden jeweils Beads ohne Template, Beads+PBS und Beads mit einem fremden Antigen verwendet.

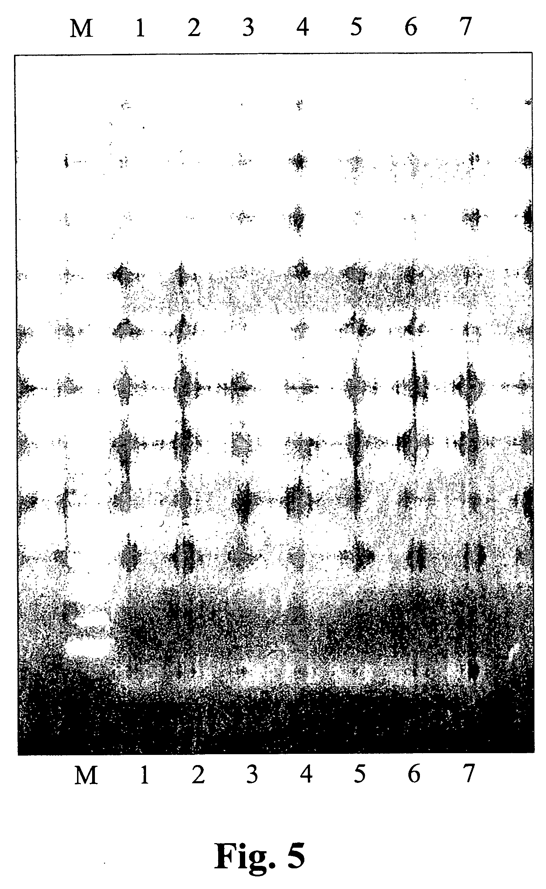

Wie in Fig. 5 zu erkennen, konnte gezeigt werden, dass nur Beads mit gebunden Legionellen als Template ein Signal ergaben (qualitativer Nachweis). Für die Quantifizierung wurde simultan eine PCR auf genomischer Legionellen DNA durchgeführt. Hierzu wurde eine definierte Menge an Genomkopien (2x10⁶) in einer Verdünnungsreihe ausgedünnt und in der PCR eingesetzt. Nach einer elektrophoretischen Auftrennung der Amplifikate konnte die Nachweisgrenze über das Agarosegel abgeschätzt werden (quantitativer Nachweis).

Fig. 5 zeigt den qualitativen und quantitativen Nachweis von Legionella pneumophila.

Auf dem oberen Agarosegel ist eine Verdünnungsreihe von einer definierten Genomkopiekonzentration ausgehend zu erkennen: Spur 1) 2x10⁶ Genome, Spur 2) 2x10⁵ Genome, Spur 3) 2x10⁴ Genome, Spur 4) 2x10³ Genome, Spur 5) 2x10² Genome, Spur 6) 2x10¹ Genome, Spur 7) Negativkontrolle ohne Genomkopien.

Auf dem unteren Agarosegel ist eine Verdünnungsreihe von Legionella pneumophila zu erkennen: Spur 8) Nachweis von ca. 2x10³ Legionellen an magnetische Beads gebunden, Spur 9) Nachweis von ca. 2x10¹ Legionellen an magnetische Beads gebunden, Spur 10) Nachweis von ca. 2x10² Legionellen an magnetische Beads gebunden, Spur 11) Nachweis von ca. 2x10⁰ Legionellen an magnetische Beads gebunden, Spur 12) Negativkonrolle ohne Legionellen an magnetische Beads gebunden, Spur 13) PCR mit Waschpuffer (PBS), Spur 14) PCR mit fremden Antigen, Spur M) DNA-Marker ein Bande entspricht 100 Basenpaaren.

Wie weiterhin der Fig. 5 zu entnehmen liegt die Nachweisgrenze bei einer Konzentration von 2000 bis 200 Zellen/12µl.

### Versuchsergebnisse zu Adenoviren

Für die Detektion der Adenoviren wurden die oben beschriebenen Primer für das Markergen Hexon verwendet. Es konnte gezeigt werden, dass mit diesen Primern mit den Legionellen vergleichbare Ergebnisse erzielt werden konnten.
## Patentansprüche

1. Verfahren zur Detektion zumindest eines Antigens, umfassend die Schritte:
a) Bereitstellen von magnetischen Beads, die mit spezifischen Antikörpern für zumindest ein zu detektierendes Antigen beschichtet sind;
b) Kontaktieren der magnetischen Beads mit einem Waschpuffer, der zumindest 8 % BSA umfasst;
c) Inkubation des Ansatzes aus Schritt b) mit einer Probe;
d) Isolierung der magnetischen Beads mittels eines magnetischen Separators; und
e) Detektion der mittels der Antikörper an die magnetischen Beads gebundenen Antigene
und wobei das zumindest eine zu detektierende Antigen ausgewählt ist aus der Gruppe bestehend aus Legionellen, Adenovirus und/oder Escherichia coli.

2. Verfahren nach Anspruch 1 , wobei der Waschpuffer zumindest 10 % BSA umfasst.

3. Verfahren nach einem der vorhergehenden Ansprüche, wobei die Isolierung der magnetischen Beads in Schritt d) zumindest einen weiteren Waschschritt umfasst.

4. Verfahren nach Anspruch 3, wobei der zumindest eine weitere Waschschritt mit demselben Waschpuffer wie in Schritt b) durchgeführt wird.

5. Verfahren nach einem der vorhergehenden Ansprüche, wobei die Detektion der an die magnetischen Beads gebundenen Antigene immunologisch und/oder mittels eines Nukleinsäureamplifikationsverfahrens durchgeführt wird.

6. Verfahren nach einem der vorhergehenden Ansprüche, wobei zumindest zwei Arten von Antikörpern die für zumindest zwei Antigene spezifisch sind verwendet werden, wobei die erste Art Antikörper für einen Mikroorganismus, ein Bakterium, Virus, Protozoe oder einen Pilz spezifisch ist und die zweite Art Antikörper für ein von diesem Mikroorganismus, Bakterium, Virus oder Pilz produziertes Toxin spezifisch ist.

7. Verfahren nach Anspruch 5, wobei der Primer die Sequenz ausgewählt aus der Gruppe bestehend aus SEQ ID NO: 1, 2, 3, 4 oder eines Homologs davon umfasst, wobei das Homolog eine Nukleinsäureidentität von zumindest 80 % aufweist.

8. Verfahren nach einem der vorhergehenden Ansprüche, wobei das Coating der Antikörper mit den magnetischen Beads über eine Biotin-Streptavidin-Bindung erfolgt.

9. Verfahren nach einem der vorhergehenden Ansprüche, wobei der Antikörper eine Bindekonstante von ungefähr 10⁵ M⁻¹ bis ungefähr 10¹⁰ M⁻¹, bevorzugter 10⁸ M⁻¹ bis ungefähr 10¹¹ M⁻¹ , noch bevorzugter 10⁹ M⁻¹ bis ungefähr 10¹² M-1 aufweist.

10. Verfahren nach einem der vorhergehenden Ansprüche, wobei die Inkubation bei Raumtemperatur oder bei einer Temperatur zwischen ≥ 20° C und ≤ 40° C, ≥ 30 C und
≤ 40° C und noch bevorzugter bei etwa 37° C durchgeführt wird.

11. Verwendung eines Waschpuffers umfassend zumindest 8% bevorzugt 10% BSA und zumindest ein Detergenz in einem Verfahren nach einem der vorhergehenden Ansprüche.

12. Kit umfassend zumindest eine Art magnetischer Beads, die mit spezifischen Antikörpern für zumindest ein zu detektierendes Antigen beschichtet sind und einen Waschpuffer, der zumindest 8% und bevorzugt 10% BSA umfasst,
wobei das zumindest eine zu detektierende Antigen ausgewählt ist aus der Gruppe bestehend aus Legionellen, Adenovirus und/oder Escherichia coli.

## Claims

1. Method of detecting at least one antigen, comprising the steps of:
a) providing magnetic beads coated with antibodies specific to at least one antigen to be detected;
b) exposing the magnetic beads to a washing buffer comprising at least 8% BSA;
c) incubating the mixture of step b) with a sample;
d) isolating the magnetic beads by means of a magnetic separator; and
e) detecting the antigens bound by means of the antibodies to the magnetic beads
and wherein the at least one antigen to be detected is selected from the group consisting of *Legionella,* adenovirus and/or *Escherichia coli.*

2. Method according to Claim 1, wherein the washing buffer comprises at least 10% BSA.

3. Method according to either of the preceding claims, wherein isolating the magnetic beads in step d) comprises at least one further washing step.

4. Method according to Claim 3, wherein the at least one further washing step is carried out using the same washing buffer as in step b).

5. Method according to any of the preceding claims, wherein detecting the antigens bound to the magnetic beads is carried out immunologically and/or by means of a nucleic acid amplification procedure.

6. Method according to any of the preceding claims, wherein at least two types of antibodies specific to at least two antigens are used, the first type of antibody being specific to a microorganism, a bacterium, virus, protozoan, or a fungus and the second type of antibody being specific to a toxin produced by said microorganism, bacterium, virus, or fungus.

7. Method according to Claim 5, wherein the primer comprises the sequence selected from the group consisting of SEQ ID NO: 1, 2, 3, 4, or a homologue thereof, the homologue having a nucleic acid identity of at least 80%.

8. Method according to any of the preceding claims, wherein the antibodies are coated with the magnetic beads via biotin-streptavidin bonding.

9. Method according to any of the preceding claims, wherein the antibody has a binding constant of from approximately 10⁵ M⁻¹ to approximately 10¹⁰ M⁻¹, more preferably 10⁸ M⁻¹ to approximately 10¹¹ M⁻¹, even more preferably 10⁹ M⁻¹ to approximately 10¹² M^{-1.}

10. Method according to any of the preceding claims, wherein incubating is carried out at room temperature or at a temperature between ≥ 20°C and ≤ 40°C, ≥ 30°C and ≤ 40°C, and even more preferably at about 37°C.

11. Use of a washing buffer comprising at least 8%, preferably 10%, BSA and at least one detergent in a method according to any of the preceding claims.

12. Kit comprising at least one type of magnetic beads coated with antibodies specific to at least one antigen to be detected and a washing buffer comprising at least 8%, preferably 10%, BSA, wherein the at least one antigen to be detected is selected from the group consisting of *Legionella,* adenovirus and/or *Escherichia coli.*

## Revendications

1. Procédé de détection d'au moins un antigène, comprenant les étapes suivantes :
a) fourniture de perles magnétiques qui sont revêtues d'anticorps spécifiques contre au moins un antigène à détecter ;
b) mise en contact des perles magnétiques avec un tampon de lavage comprenant au moins 8 % de BSA ;
c) incubation de la masse réactionnelle de l'étape b) avec un échantillon ;
d) isolement des perles magnétiques à l'aide d'un séparateur magnétique ; et
e) détection des antigènes liés aux perles magnétiques à l'aide des anticorps,
et dans lequel le ou les antigènes à détecter sont choisis dans le groupe consistant en les légionnelles, les adénovirus et/ou Escherichia coli.

2. Procédé selon la revendication 1, dans lequel le tampon de lavage comprend au moins 10 % de BSA.

3. Procédé selon l'une des revendications précédentes, dans lequel l'isolement des perles magnétiques dans l'étape d) comprend au moins une étape de lavage supplémentaire.

4. Procédé selon la revendication 3, dans lequel le ou les étapes de lavage supplémentaires sont mises en oeuvre avec le même tampon de lavage que dans l'étape b) .

5. Procédé selon l'une des revendications précédentes, dans lequel la détection des antigènes liés aux perles magnétiques est réalisée d'une manière immunologique et/ou par un procédé d'amplification d'acides nucléiques.

6. Procédé selon l'une des revendications précédentes, **caractérisé en ce qu'**on utilise deux-types d'anticorps, qui sont spécifiques d'au moins deux antigènes, le premier type d'anticorps étant spécifique d'un microorganisme, d'une bactérie, d'un virus, d'un protozoaire ou d'un champignon, et le deuxième type d'anticorps étant spécifique d'une toxine produite par ce microorganisme, cette bactérie, ce virus ou ce champignon.

7. Procédé selon la revendication 5, dans lequel l'amorce comprend la séquence choisie dans le groupe consistant en SEQ ID NO:1, 2, 3 ou 4 ou un homologue de celle-ci, l'homologue présentant une identité d'acides nucléiques d'au moins 80 %.

8. Procédé selon l'une des revendications précédentes, dans lequel le revêtement des anticorps a lieu avec les perles magnétiques par l'intermédiaire d'une liaison biotine-streptavidine.

9. Procédé selon l'une des revendications précédentes, dans lequel l'anticorps présente une constante de liaison d'environ 10⁵ M⁻¹ à environ 10¹⁰ M⁻¹, de préférence de 10⁸ M⁻¹ à environ 10¹¹ M⁻¹, d'une manière encore plus préférée de 10⁹ M⁻¹ à environ 10¹² M⁻¹.

10. Procédé selon l'une des revendications précédentes, dans lequel l'incubation est mise en oeuvre à la température ambiante ou à une température comprise entre ≥ 20°C et ≤ 40°C, ≥ 30°C et ≤ 40°C, et d'une manière encore plus préférée à environ 37°C.

11. Utilisation d'un tampon de lavage comprenant au moins 8 %, de préférence 10 % de BSA, et au moins un détergent, dans un procédé selon l'une des revendications précédentes.

12. Trousse comprenant au moins un type de perles magnétiques, qui sont revêtues d'anticorps spécifiques contre au moins un antigène à détecter, et un tampon de lavage, qui comprend au moins 8 % et de préférence 10 % de BSA,
dans laquelle le ou les antigènes à détecter sont choisis dans le groupe consistant en les légionnelles, les adénovirus et/ou Escherichia coli.
